# EUROPEAN PATENT SPECIFICATION

(11) **EP 3 500 213 B1**
(45) Date of publication and mention of the grant of the patent: **27.11.2019**
(21) Application number: 16778449.5
(22) Date of filing: 22.08.2016
(51) Int. Cl.: A61F 2/08, A61F 2/40, A61B 17/11, A61F 2/00

(54) **ARTIFICIAL LIGAMENT FOR ACROMIOCLAVICULAR LUXATION**
KÜNSTLICHES BAND FÜR AKROMIOKLAVIKULÄRE LUXATION
LIGAMENT ARTIFICIEL POUR LUXATION ACROMIO-CLAVICULAIRE

(43) Date of publication of application: 26.06.2019
(73) Proprietor: Calvosa, Giuseppe, 56123 Pisa (IT)
(72) Inventor: TENUCCI, Miria, 55100 Lucca (IT); GALGANI, Matteo, 50052 Certaldo (FI) (IT); CALVOSA, Giuseppe, 56123 Pisa (PI) (IT)
(74) Representative: Papa, Elisabetta
(86) International application number: PCT/IB2016/055001
(87) International publication number: WO 2018/037257

(56) References cited:
- WO-A1-02/30324
- DE-A1- 3 710 587
- FR-A1- 2 810 877
- GB-A- 2 474 866
- US-A1- 2002 123 750
- US-A1- 2008 188 936

## Description

### Technical field of the invention

The present invention relates to an artificial ligament for a shoulder dislocation, in particular for an acromioclavicular luxation.

The present description also relates to a surgical method for implanting said artificial ligament.

### Background

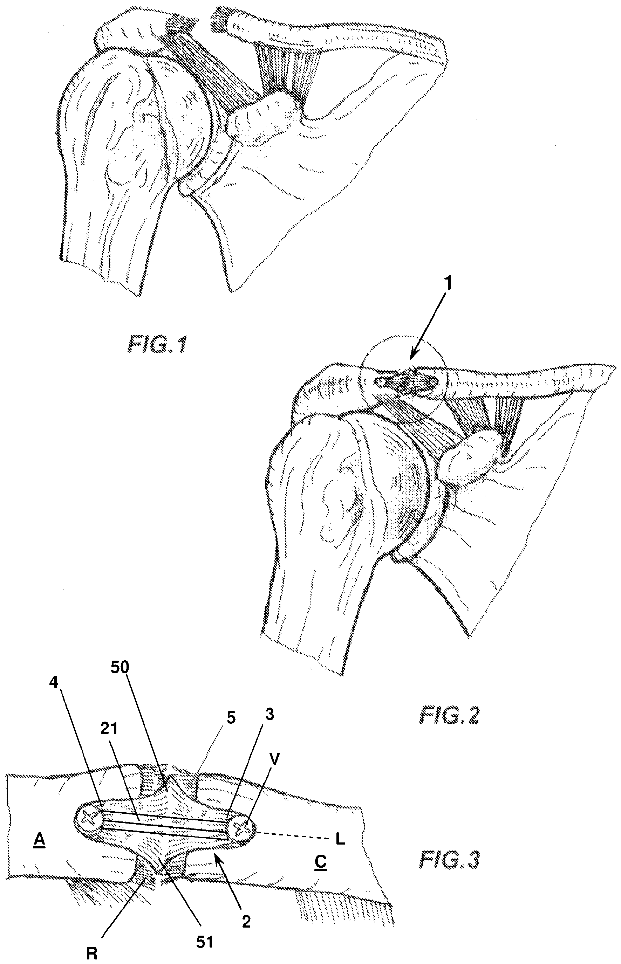

An acromioclavicular luxation is a traumatic pathology affecting the joint and is exemplified, in general terms, in Figure 1. This dislocation involves the articular capsule, the acromioclavicular and coracoclavicular ligaments and, in the worst cases, the deltoid and trapezius muscles. It represents about 12% of all shoulder dislocations, but occurs most frequently in young athletes. In fact, the traumatic mechanism is that associated with a sideways fall and its main causes are sports injuries - for example in skiing, rugby and cycling - and road accidents, in particular those involving motorcyclists.

The classification made by Allman (Allman FL Jr.: "Fractures and ligamentous injuries of the clavicle and its articulation." - J Bone Joint Surg 1967 49-A, 77-784) divides up acromioclavicular luxations into three types:
▪ type I - distortion of the joint, affecting the articular capsule, without involvement of the acromioclavicular and coracoclavicular ligaments;
▪ type II - involvement of the acromioclavicular ligaments with consequent subluxation of the joint;
▪ type III - lesion of the acromioclavicular and coracoclavicular ligaments with consequent dislocation of the joint (distal articular facet of the clavicle dislocated by at least 75% with respect to the acromial articular facet).

Rockwood's classification (Rockwood CA jr, Matsen F.A. III: "The Shoulder." - W B Saunders, Philadelphia, 1990) defines instead six types of lesion, in particular:
▪ type I - distortional trauma of the acromioclavicular joint;
▪ type II - lesion of the acromioclavicular ligaments, without breakage;
▪ type III - lesion of the acromioclavicular and coracoclavicular ligaments, increase in the coracoclavicular space by more than 25%, deltoid and trapezius muscles detached;
▪ type IV - lesion of the acromioclavicular and coracoclavicular ligaments, the clavicle is dislocated at the rear inside the trapezius muscle and this increases the pain;
▪ type V - lesion of the acromioclavicular and coracoclavicular ligaments, major increase in the coracoclavicular space (more than 100% of the nominal value), deltoid and trapezius muscles detached from half of the distal clavicle;
▪ type VI - very rare, the clavicle is dislocated underneath the coracoid or acromion.

As regards treatment of the aforementioned types of lesions, there exist conservative approaches, namely those based on healing/regeneration of the physiological ligament, and surgical techniques for replacement of the ligament with an artificial or indigenous element.

One of the most widely used conservative techniques envisages the use of an acromioclavicular plate for fixing the joint. This plate does not interfere with healing of the ligament and allows a minimally invasive approach and precocious movements. However, the plate requires a second operation for removal.

Other surgeons use a so-called coracoclavicular screw, namely a spongy bone screw with a diameter of between 4 and 5 mm, positioned perpendicularly between clavicle and coracoid. This screw has a mechanical function similar to the main ligaments and does not require a high level of skill on the part of the surgeon. However, the most frequent complication associated with it is the reduction loss and it requires a second surgical operation for removal.

However, hitherto it has not been possible to identify an ideal treatment method. In fact, none of the treatments proposed in the prior art results in an optimum compromise between the different therapeutic requirements, in particular quality and range of movement permitted for the joint, operating time, simplicity of surgical technique and minimization of recurrences.

Document FR2810877 describes an artificial ligament according to the preamble of claim 1.

### Summary of the invention

The drawbacks mentioned above with reference to the prior art are overcome in the present invention by providing an acromioclavicular artifical ligament which is able to optimize the different operating requirements.

The artificial ligament according to the invention is defined in claim 1.

Preferred characteristic features of the invention are object of the dependent claims.

The invention offers significant advantages, some of which are summarized below.
▪ It is possible to act directly on the acromioclavicular joint by means of reduction and stabilization of the dislocation.
▪ It is possible to act using a superior surgical approach with a "shoulder incision" directly on the lesion and with minimum impact from an aesthetic point of view.
▪ It is possible to reconstruct anatomically the acromioclavicular joint albeit with an artificial ligament and thus maintain the physiological micro-motility of the acromioclavicular joint. Maintaining this motility in the physiological range is essential for the biomechanics of the shoulder and correct functioning thereof.
▪ It is possible to perform the operation by means of a mini-invasive and/or percutaneous procedure.

Other advantages, characteristic features and modes of use of the present invention will become clear from the following detailed description of some embodiments thereof, provided by way of a non-limiting example.

### Brief description of the figures

Reference will be made to the figures of the accompanying drawings, in which:
▪ Figure 1, already mentioned above, shows an example of a possible case of acromioclavicular luxation;
▪ Figure 2 shows a schematic front view of a preferred embodiment of the artifical ligament according to the invention, in a configuration implanted in a patient; and
▪ Figure 3 shows a view, on a larger scale, of the ligament shown in Figure 2.

The thicknesses, the dimensions and the curvatures shown in the figures mentioned above must be understood as being purely exemplary and are generally on a larger scale and not necessarily shown in proportion.

In particular, the articular segments, especially the acromion, have been shown in extremely schematic form.

### Detailed description of preferred embodiments

With reference to Figures 2 and 3, an artifical ligament according to a preferred embodiment of the invention is denoted overall by 1. The ligament 1 is configured to be used for surgical treatment of a shoulder dislocation, in particular for replacement of a physiological acromioclavicular ligament or for assisting it.

The artificial ligament 1 has, or is defined by, an elongated main body 2 made at least partly of a biocompatible material. For example, the main body 2 may be made of polyethylene, in particular the material known as Dyneema® (*Gel Spun Polyethylene*), or other material.

The main body 2 extends principally along a line or direction L, between a first longitudinal end 3 and a second longitudinal end 4.

The two longitudinal ends 3 and 4 are designed or configured to be secured to the clavicle C and to the acromion A, respectively. In a preferred variation of embodiment, the two ends are interchangeable, in the sense that each of them may be equally well fixed onto the clavicle or onto the acromion.

In the embodiment considered here, the main body 2 is formed at least partly by a plurality of thread-like elements arranged alongside each other and/or interwoven.

The main body 2 comprises an elongated central portion 21, in particular extending along the line L. The central portion 21 is continuous or substantially continuous between said longitudinal ends 3 and 4. In particular, between these two ends 3 and 4, the central portion 21 is formed by one or more elements, which are thread-like, extending without interruptions or cuts.

The main body 2 also has an enlarged portion 5, arranged in a longitudinally intermediate position, albeit not necessarily at a centre line, between the two ends 3 and 4. The enlarged position 5 is understood as defining an anchoring region for one or more residual physiological ligament elements R. In the present example, the enlarged portion 5 defines a pair of anchoring regions denoted by 50 and 51 and arranged bilaterally on the central portion 21.

In the embodiment considered here, the enlarged portion 5 has a substantially lozenge or rhomboid shape and said anchoring regions 50 and 51 are each defined at or in the vicinity of an angle of the lozenge or rhomboid. In particular, these regions 50 and 51 are associated with two opposite angles of the lozenge or rhomboid, which in the present example correspond to a smaller diagonal. The angles associated with a larger diagonal D2 are understood as being arranged along the central portion 21, namely along the line or direction L.

Variations of embodiments may envisage an enlarged portion with an again substantially quadrangular form or, not according to the present invention, with an at least partially rounded perimetral form.

According to the present invention, the anchoring regions are each shaped as an angle or arrowhead.

In the preferred configuration described, the central portion 21 defines a continuous ligamentous element and the enlarged portion 5 extends laterally with respect to the central portion 21.

On the basis of preferred variations of embodiment, the central portion 21 of the main body 2 has a geometrical form which is substantially cylindrical or in any case with a solid cross-section. According to other variants, the enlarged portion 5 and/or the entire main body 2 may have a flat or substantially flat form.

As regards the method for implanting the artificial ligament 1, a possible technique is described below.

Firstly, the ligament is introduced into a central hole suitably formed on the body of the acromion. Then a hole is made in the centre of the clavicle in the lateral third thereof and the ligament is inserted inside the hole. This is followed by reduction of the dislocation and then the two longitudinal ends 3 and 4 of the ligament 1 may be fixed on the respective bone segment by means of interference screws V. The latter may be for example made of titanium or also at least partly of reabsorbable material.

Residual physiological ligament portions R may be sutured, or fixed using another technique, at the anchoring regions 50 and 51. In this way, the acromioclavicular joint is further reinforced and its original anatomical form is thus reestablished.

The present invention has been described hitherto with reference to preferred embodiments thereof. It is to be understood that other embodiments relating to the same inventive idea may exist, as defined by the scope of protection of the claims reported herein below.

## Claims

1. An acromioclavicular artificial ligament (1),
which artificial ligament (1) comprises an elongated main body (2) made of biocompatible material,
which main body (2) has a first longitudinal end (3) apt to be secured to the clavicle and a second longitudinal end (4) apt to be secured to the acromion,
wherein said main body (2) has an elongated central portion (21) defining a continuous ligamentous element between said first (3) and second (4) longitudinal end and formed by a plurality of thread-like elements,
wherein said main body (2) further has an enlarged portion (5) arranged in a longitudinally intermediate position and
**characterized in that** said plurality of thread-like elements are arranged alongside each other and extending without interruptions or cuts and **in that** said enlarged portion (5) defines a pair of anchoring regions (50, 51) for anchoring a residual physiological ligament element, which anchoring regions (50, 51) extend bilaterally with respect to said elongated central portion (21), wherein said anchoring regions (50, 51) are each shaped as an angle or arrowhead.

2. The artificial ligament (1) according to claim 1, wherein said enlarged intermediate portion (5) extends bilaterally on said main body (2).

3. The artificial ligament (1) according to claim 1 or 2, wherein said enlarged intermediate portion (5) has a substantially lozenge, rhomboid or quadrangular shape.

4. The artificial ligament (1) according to any one of the preceding claims, wherein said enlarged intermediate portion (5) is substantially flat.

5. The artificial ligament (1) according to any one of the preceding claims, made at least in part of polyethylene or other biocompatible material.

## Patentansprüche

1. Künstliches akromioklavikuläres Band (1),
wobei das künstliche Band (1) umfasst: ein längliches Hauptteil (2), das aus einem biokompatiblen Material besteht,
wobei das Hauptteil (2) über ein erstes Längsende (3) verfügt, das geeignet ist, an der Klavikula befestigt zu werden, und über ein zweites Längsende (4) verfügt, das geeignet ist, am Acromion befestigt zu werden, wobei das Hauptteil (2) über einen länglichen zentralen Abschnitt (21) verfügt, der ein kontinuierliches Bandelement zwischen dem ersten (3) und zweiten (4) Längsende definiert und durch eine Mehrzahl von fadenförmigen Elementen gebildet wird,
wobei das Hauptteil (2) weiterhin über einen vergrößerten Abschnitt (5) verfügt, der in Längsrichtung eine Zwischenposition einnimmt, und
**dadurch gekennzeichnet, dass** die Mehrzahl von fadenförmigen Elementen nebeneinander angeordnet sind und sich ohne Unterbrechungen oder Schnitte erstrecken und somit der vergrößerte Abschnitt (5) ein Paar von Ankerbereichen (50, 51) zum Verankern eines restlichen physiologischen Bandelements definiert, wobei sich die Ankerbereiche (50, 51) bezüglich des länglichen zentralen Abschnitts (21) bilateral erstrecken, wobei die Ankerbereiche (50, 51) jeweils als ein Winkel oder eine Pfeilspitze geformt sind.

2. Künstliches Band (1) gemäß Anspruch 1, wobei sich der vergrößerte Zwischenabschnitt (5) bilateral auf dem Hauptteil (2) erstreckt.

3. Künstliches Band (1) gemäß Anspruch 1 oder 2, wobei der vergrößerte Zwischenabschnitt (5) im Wesentlichen rautenförmig, rhombenförmig oder viereckig ist.

4. Künstliches Band (1) gemäß einem der vorangehenden Ansprüche, wobei der vergrößerte Zwischenabschnitt (5) im Wesentlichen flach ist.

5. Künstliches Band (1) gemäß einem der vorangehenden Ansprüche, das mindestens zum Teil aus Polyethylen oder einem anderen biokompatiblen Material besteht.

## Revendications

1. Ligament artificiel acromio-claviculaire (1),
lequel ligament artificiel (1) comprend un corps principal allongé (2) réalisé avec un matériau biocompatible,
lequel corps principal (2) a une première extrémité longitudinale (3) apte à être fixée à la clavicule et une seconde extrémité longitudinale (4) apte à être fixée à l'acromion,
dans lequel ledit corps principal (2) a une partie centrale allongée (21) définissant un élément ligamenteux continu entre lesdites première (3) et seconde (4) extrémités longitudinales et formé par une pluralité d'éléments en forme de fil,
dans lequel ledit corps principal (2) a en outre une partie agrandie (5) agencée dans une position longitudinalement intermédiaire, et
**caractérisé en ce que** ladite pluralité d'éléments en forme de fil sont agencés les uns le long des autres et s'étendant sans interruptions ni coupes et **en ce que** ladite partie agrandie (5) définit une paire de régions d'ancrage (50, 51) pour s'ancrer à un élément de ligament physiologique résiduel, lesdites régions d'ancrage (50, 51) s'étendent de manière bilatérale par rapport à ladite partie centrale allongée (21), dans lequel lesdites régions d'ancrage (50, 51) sont chacune formées comme un angle ou une tête de flèche.

2. Ligament artificiel (1) selon la revendication 1, dans lequel ladite partie intermédiaire agrandie (5) s'étend de manière bilatérale sur ledit corps principal (2).

3. Ligament artificiel (1) selon la revendication 1 ou 2, dans lequel ladite partie intermédiaire agrandie (5) a sensiblement une forme losange, rhomboïde ou quadrangulaire.

4. Ligament artificiel (1) selon l'une quelconque des revendications précédentes, dans lequel ladite partie intermédiaire agrandie (5) est sensiblement plate.

5. Ligament artificiel (1) selon l'une quelconque des revendications précédentes, réalisé au moins en partie avec du polyéthylène ou avec un autre matériau biocompatible.
